# NOUVEAU FASCICULE DE BREVET EUROPEEN

(11) **EP 0 797 979 B2**
(45) Date de publication et mention de la décision concernant l'opposition: **25.02.2009**
(45) Mention de la délivrance du brevet: 09.09.1998
(21) Numéro de dépôt: 97400726.2
(22) Date de dépôt: 28.03.1997
(51) Int. Cl.: A61K 8/73, A61K 8/81, A61Q 5/04

(54) **Composition cosmétique comprenant un polymère fixant et un amidon amphotère**
Ein bildendes Polymer und eine amphoterische Stärke enthaltende kosmetische Zusammensetzung
Cosmetic composition containing a binding polymer and an amphoteric starch

(30) Priorité: 05.04.1996 FR 9604367
(43) Date de publication de la demande: 01.10.1997
(73) Titulaire: L'ORÉAL, 75008 Paris (FR)
(72) Inventeur: Dupuis, Christine, 75018 Paris (FR)
(74) Mandataire: Dossmann, Gérard

(56) Documents cités:
- EP-A- 0 689 829
- US-A1- 5 482 704

## Description

La présente invention a trait à l'utilisation d'une composition cosmétique pour le traitement des cheveux, comprenant au moins un polymère fixent et au moins un amidon amphotère.

Les compositions de maintien ou de mise en forme des cheveux contenant dans leur formulation des polymères de coiffage (polymère fixants) présentent généralement l'inconvénient de rendre difficile le démêlage, le recoiffage ou le brossage des cheveux, en particulier pendant un brushing. Les cheveux traités avec ces polymères fixants sont généralement rêches et ont un touche non naturel.

On connaît par EP 689 829 l'association de dérivés d'amidon dans des compositions cosmétiques. Ce document décrit la mise en oeuvre de ces composés en tant qu'agents épaississants ou stabilisants.

L'association de dérivés siliconés avec des polymères fixants est connue dans des compositions cosmétiques pour le maintien et/ou la fixation de la coiffure. Il a été constaté que ces dérivés siliconés améliorent les propriétés de démêlage, de douceur et de brillance des cheveux traités à l'aide de ces compositions. Cependant, les dérivés siliconés ne sont pas favorables aux propriétés coiffantes des compositions contenant des polymères fixants.

En particulier, on recherche des compositions permettant de modifier la texture des cheveux, c'est-à-dire des compositions qui ramollissent les cheveux lors de l'application sur les cheveux mouillés (propriété d'émollience) et qui permettent également un démêlage très facile. Ces propriétés sont en général apportés par des cations, mais les cheveux une fois séchés sont mous et alourdis par ces compositions. La coiffure n'a pas de volume.

Le but de la présente invention est donc de proposer des compositions permettant de fixer et/ou de mettre en forme la coiffure, ces compositions devant avoir de bonnes propriétés de fixation et/ou de tenue dans le temps et apporter d'excellents propriétés cosmétiques telles que l'émollience, la douceur, le démêlage et le toucher.

Or, la demanderesse a découvert qu'en utilisant des compositions contenant un polymère fixant en associant avec au moins un amidon amphotère dans un milieu cosmétiquement acceptable, on obtient d'excellentes propriétés cosmétiques telles que la douceur, le démêlage et le toucher tout en ayant des propriétés coiffantes et/ou fixantes synergiques.

Le pouvoir fixant des compositions selon l'invention est supérieur à celui des compositions ne contenant qu'un seul des deux composés.

La présente invention a donc pour objet l'utilisation d'une composition cosmétique comprenant, dans un milieu cosmétiquement acceptable, au moins un polymère fixant et au moins un amidon amphotère, pour la fixation ou la mise en forme des cheveux.

Dans le cadre de la présente demande, on entend par compositions cosmétiques pour le maintien de la coiffure toute composition ayant pour fonction de fixer temporairement la forme de la coiffure, comme par exemple les laques et sprays de coiffage, les gels et les mousses de coiffage. Par pouvoir fixant d la composition, on désigne l'aptitude de cette dernière à donner aux cheveux une cohésion de telle sorte que la mise en forme initiale de la coiffure soit conservée. Par polymère fixant, on entend tout polymère ayant pour fonction de fixer la forme de la coiffure.

Selon la présente invention, les amidons amphotères et les polymères amphotères peuvent être éventuellement zwittérioniques.

Les amidons amphotères utilisables selon l'invention contiennent un ou plusieurs groupements anioniques et un ou plusieurs groupements cationiques. Les groupements anioniques et cationiques peuvent être liés au même site réactif de la molécule d'amidon ou à des sites réactifs différents; de préférence ils sont liés au même site réactif.

Les groupements anioniques peuvent être de type carboxylique, phosphate ou sulfate et de préférence carboxylique. Les groupements cationiques peuvent être de type amine primaire, secondaire, tertiaire ou quaternaire.

Les amidons utilisables selon l'invention sont choisis de préférence parmi les composés de formules suivantes : 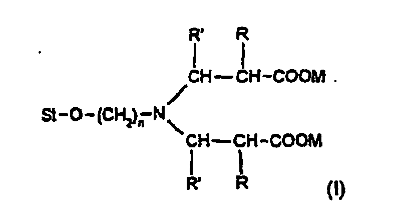 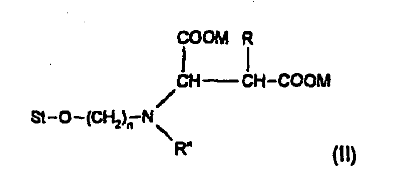 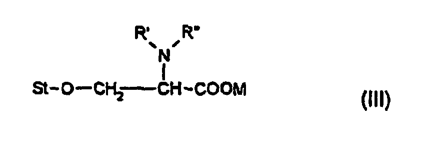 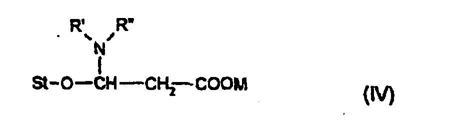 formules dans lesquelles:
St-O représente une molécule d'amidon,
R, identique ou différent, représente un atome d'hydrogène ou un radical méthyle,
R', identique ou différent, représente un atome d'hydrogène, un radical méthyle ou un groupement -COOH,
n est un entier égal à 2 ou 3,
M, identique ou différent, désigne un atome d'hydrogène, un métal alcalin ou alcalinoterreux tels que Na, K, Li, NH₄, ou une amine organique,
R" représente un atome d'hydrogène ou un radical alkyle ayant de 1 à 18 atomes de carbone.

Ces composés sont notamment décrits dans les brevets US 5,455,340 et US 4,017,460.

Les molécules d'amidons peuvent être issues de toutes les sources végétales d'amidon telles que notamment le maïs, les pommes de terre, l'avoine, le riz, le tapioca, le sorgho, l'orge ou le blé. On peut également utiliser les hydrolysats des amidons cités ci-dessous. L'amidon est de préférence issu de la pomme de terre.

On utilise particulièrement les amidons de formules (I) ou (II). On utilise particulièrement les amidons modifiés par de l'acide 2-chloroéthyl aminodipropionique, c'est à dire les amidons de formule (I) ou (II) dans lesquelles R, R' R" et M représentant un atome d'hydrogène et n'est égal à 2.

Selon l'invention, on peut utiliser tout polymère fixant connu en soi. On peut utiliser en particulier un polymère fixant choisi parmi les polymères anioniques, cationiques, amphotères, non ioniques et leurs mélanges. Les polymères fixants peuvent être utilisés sous forme solubilisée ou sous forme de dispersions de particules solides de polymère.

Les polymères fixants cationiques utilisables selon la présente invention sont de préférence choisis parmi les polymères comportant des groupements amine primaire, secondaire, tertiaire et/ou quaternaire faisant partie de la chaîne polymère ou directement reliés à celle-ci, et ayant un poids moléculaire compris entre 500 et environ 5.000.000 et de préférence entre 1000 est 3.000.000.

Parmi ces polymères, on peut citer plus particulièrement les polymères cationiques suivants:
(1) les homopolymères ou copolymères dérivés d'esters ou d'amides acryliques ou méthacryliques et comportant au moins un des motifs de formules suivantes: 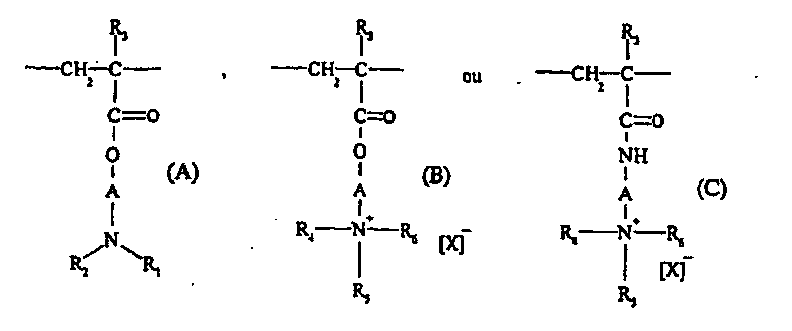 dans lesquelles:
   R₃ désigne un atome d'hydrogène ou un radical CH₃;
   A est un groupe alkyle linéaire ou ramifié de 1 à 6 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle;
   R₁ et R₂ représentent hydrogène ou un groupe alkyle ayant de 1 à 6 atomes de carbone;
   X désigne un anion méthosulfate ou un halogénure tel que chlorure ou bromure.

   Les copolymères de la famille (1) contiennent en outre un ou plusieurs motifs dérivant de comonomères pouvant être choisis dans la famille des acrylamides, methacrylamides, diacétones acrylamides, acrylamides et méthacrylamides substitués sur l'azote par des alkyles inférieurs, des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer:
   - les copolymères d'acrylamide et de diméthylaminoéthyl méthacrylate quaternisé au sulfate de diméthyle ou avec un halogénure de diméthyle tels que celui vendu sous la dénomination HERCOFLOC par la société HERCULES,
   - les copolymères d'acrylamide et de chlorure de méthacryloyloxyéthyltriméthylammonium décrit par exemple dans la demande de brevet EP-A-080976 et vendus sous la dénomination BINA QUART P 100 par la société ClBA GEIGY,
   - le copolymère d'acrylamide et de méthosulfate de méthacryloyloxyéthyl; triméthylammonium vendu sous la dénomination RETEN par la société HERCULES,
   - les copolymères vinylpyrrolidone/acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 93T. Ces polymères sont décrits en détail dans les brevets français 2.077.143 et 2.393.573,
   - les terpolymères méthacrylate de diméthyl amino éthyle/ vinylcaprolactame/ vinylpyrrolidone tel que le produit vendu sous la dénomination GAFFIX VC 713 par la société ISP,
   - et le copolymère vinylpyrrolidone / méthacrylamide de diméthylaminopropyle quaternisé tel que le produit vendu sous la dénomination "GAFQUAT HS 100" par la société ISP.
(2) les polysaccharides quaternisés décrits plus particulièrement dans les brevets américains 3.589.578 et 4.031.307 tel que les gommes de guar contenant des groupements cationiques trialkylammonium.
   De tels produits sont commercialisés notamment sous les dénominations commerciales de JAGUAR C13 S. JAGUAR C 15, JAGUAR C 17 par la société MEYHALL.
(3) les copolymères quaternaires de vinylpyrrolidone et de vinylimidazole;
(4) les chitosanes ou leurs sels;
   les sels utilisables sont en particulier les acétate, lactate, glutamate, gluconate ou le pyrrolidone carboxylate chitosane.
   Parmi ces composés, on peut citer le chitosane ayant un taux de désacétylation de 90,5% en poids vendu sous la dénomination KYTAN BRUT STANDARD par la société ABER TECHNOLOGIES, le pyrrolidone carboxylate de chitosane vendu sous la dénomination KYTAMER PC par la société AMERCHOL.
(5) les dérivés de cellulose cationiques tels que les copolymères de cellulose ou de dérivés de cellulose greffés avec un monomère hydrosoluble comportant un ammonium quaternaire, et décrits notamment dans le brevet US 4 131 576, tels que les hydroxyalkyl celluloses, comme les hydroxyméthyl-, hydroxyéthyl- ou hydroxypropyl- celluloses greffées notamment avec un sel de méthacryloyloxyéthyl triméthylammonium, de méthacrylamidopropyl trimétrylammonium, de diméthyl-diallylammonium.

Les produits commercialisés répondant à cette définition sont plus particulièrement les produits vendus sous la dénomination "CELQUAT L 200" et "CELQUAT H 100" par la Société National Starch.

Les polymères fixants anioniques généralement utilisés sont des polymères comportant des groupements dérivés d'acide carboxylique, sulfonique ou phosphorique et ont un poids moléculaire compris entre environ 500 et 5.000.000.
1) Les groupements carboxyliques sont apportés par des monomères mono ou diacides carboxyliques insaturés tels que ceux répondant à la formule : 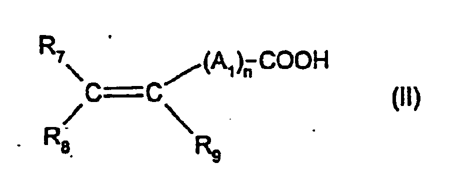 dans laquelle n est un nombre entier de 0 à 10. A₁ désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₇ désigne un atome d'hydrogène, un groupement phényle ou benzyle, R₃ désigne un atome d'hydrogène, un groupement alkyle inférieur ou carboxyle, R₉ désigne un atome d'hydrogène, un groupement alkyle inférieur, un groupement -CH₂-COOH, phényle ou benzyle: Dans la formule précitée un radical alkyle intérieur désigne de préférence un groupement ayant 1 à 4 atomes de carbone et en particulier, méthyle et éthyle.
   Les polymères fixants anioniques à groupements carboxyliques préférés selon l'invention sont :
   A) Les homo- ou copolymères d'acide acrylique ou méthacrylique ou leurs sels en particulier les produits vendus sous les dénominations VERSICOL E ou K par la société ALLIED COLLOID et ULTRAHOLD par la société BASF. Les copolymères d'acide acrylique et d'acrylamide vendus sous la forme de leur sel de sodium sous les dénominations RETEN 421, 423 ou 425 par la société HERCULES, les sels de sodium des acides polyhydroxycarboxyliques.
   B) Les copolymères des acides acrylique ou méthacrylique avec un monomère monoéthylènique tel que l'éthylène, le styrène, les esters vinyliques, les esters d'acide acrylique ou méthacrylique, éventuellement greffés sur un polyalkylène glycol tel que le polyéthylène glycol et éventuellement réticulés. De tels polymères sont décrits en particuler dans le brevet français 1.222.944 et la demande allemande 2.330.956, les copolymères de ce type correspondant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER par la société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀ par exemple de lauryle tel que celui vendu par la société ISP sous la dénomination ACRYLIDONE LM et les terpolymères acide méthacrylique/ acrylate d'éthyle/ acrylate de tertiobutyle tel que le produit vendu sous la dénomination LUVIMER 100 P par la société BASF.
   C) les copolymères dérivés d'acides crotonique tels que ceux comportant dans leur chaîne des motifs acétate ou propionate de vinyle et éventuellement d'autres monomères tels que esters allylique ou méthallylique, éther vinylique ou ester vinylique d'un acide carboxylique saturé linéaire ou ramifié longue chaîne hydrocarbonée tels que ceux comportant au moins 5 atomes de carbone, ces polymères pouvant éventuellement être greffées et réticulées ou encore un ester vinylique, allylique ou méthallylique d'un acide carboxylique α-ou β-cyclique. De tels polymères sont décrits entre autres dans les brevets français 1.222.944, 1.580.545, 2.265.782, 2.265.781, 1.564.110 et 2.439.798. Des produits commerciaux entrant dans cette classe sont les résines 28-29-30, 26-13-14 et 28-13-10 vendues par la société NATIONAL STARCH.
   D) les copolymères dérivés d'acides ou d'anhydrides carboxyliques monoinsaturés en C₄-C₈ choisis parmi :
      - les copolymères comprenant (i) un ou plusieurs acides ou anhydrides maléique, tumarique, itaconique et (ii) au moins un monomère choisis parmi les esters vinyliques, les éthers vinyliques, les halogénures, vinyliques, les dérivés phénylvinyliques, l'acide acrylique et ses esters, les fonctions anhydrides de ces copolymères étant éventuellement monoestérifiées ou monoamidifiées. ; De tels polymères sont décrits en parliculier dans les brevets US 2.047.398, 2.723.248, 2.102.113, le brevet GB 839.805 et notamment ceux vendus sous les dénominations GANTREZ AN ou ES par la société ISP.
      - les copolymères comprenant (i) un ou plusieurs anhydrides maléiques, citraconiques, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique vinylpyrrolidone dans leur chaîne,

      les fonctions anhydrides de ces copolymères étant éventuellement monoestérifiées ou monoamidifiées.
      Ces polymères sont par exemple décrits dans les brevets français 2.350.384 et 2.357.241 de la demanderesse.
   E) les polyacrylamides comportant des groupements carboxylates. Les polymères comprenant les groupements sulfoniques sont des polymères comportant des motifs vinylsulfonique, styrène sulfonique, naphtalène sulfonique ou acrylamido alkylsulfonique.

   Ces polymères peuvent être notamment choisis parmi :
   - les sels de l'acide polyvinylsulfonique ayant un poids moléculaire compris entre environ 1.000 et 100.000 ainsi que les copolymères avec un comonomère insaturé tel que les acides acrylique ou méthacrylique et leurs esters ainsi que l'acrylamide ou ses dérivés, les éthers vinyliques et la vinylpyrrolidone.
   - les sels de l'acide polystyrène sulfonique les sels de sodium ayant un poids moléculaire d'environ 500.000 et d'environ 100.000 vendus respectivement sous les dénominations Flexan 500 et Flexan 130 par National Starch. Ces composés sont décrits dans le brevet FR 2.198.719.
   - les sels d'acides polyacrylamide sulfoniques ceux mentionnés dans le brevet US 4.128.631 et plus particulièrement l'acide polyacrylamidoéthylpropane sulfonique vendu sous la dénomination COSMEDIA POLYMER HSP 1180 par Henkel.

Selon l'invention, les polymères fixants anioniques sont de préférence choisis parmi les copolymères d'acide acrylique tels que le terpolymère acide acrylique/acrylate d'éthyle/N-tertiobutylacrylamide vendu sous la dénomination ULTRAHOLD STRONG par la société BASF, les copolymères dérivés d'acide crotonique tels que les terpolymères acétate de vinyle / tertio-bytyl benzoate de vinyle / acide crotonique et les terpolymères acide crotonique / acétate de vinyle/néododécanoate de vinyle vendus sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que le copolymère méthylvinyléther/anhydride maléique mono estérifié vendu sous la dénomination GANTREZ ES 425 par la société ISP, les copolymères d'acide méthacrylique et de méthacrylate de methyle vendus sous la dénomination EUDRAGIT L par la société ROHM PHARMA, le copolymère d'acide méthacrylique et d'acrylate d'éthyle vendu sous la dénomination LUVIMER MAEX ou MAE par la société BASF, le copolymère acétate de vinylelacide crotonique vendu sous la dénomination LUVISET CA 66 par la société BASF et la copolymère acétate de vinyle/acide crotonique greffé par du polyéthylèneglycol sous la dénomination ARISTO-FLEX A par la société BASF.

Les polymères fixants anioniques les plus particulièrement préférés sont choisis parmi le copolymère méthyvinyléther/anhydride maléique mono estérifié vendu sous la dénomination GANTREZ ES 425 par la société ISP, le terpolymère acide acrylique/acrylate d'éthyle/N-tertiobutylacrylamide vendu sous la dénomination ULTRAHOLD STRONG par la société BASF, les copolymères d'acide méthacrylique et de méthacrylate de méthyle vendus sous la dénomination EUDRAGIT L par la société ROHM PHARMA, les terpolymères acétate de vinyle/tertiobutyl benzoate de vinyle/acite crotonique et les terpolymères acide crotonique/acétate de vinyle/néododécanoate de vinyle vendus sous la dénomination Résine 28-29-30 par la société NATIONAL STARCH, le copolymère d'acide méthacrylique et d'acrylate d'éthyle vendu sous la dénomination LUVIMER MAEX OU MAE par la société BASF, le terpolymère de vinylpyrrolidone / acide acrylique/methacrylate de lauryle vendu sous la dénomination ACRYLIDONE LM par la société ISP.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques ou bien B et C peuvent désigner des groupements dérivant de monomères zwittérioniques de carboxybétaïnes ou de sulfobétaïnes; B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné ou bien B et C font partie d'une chaîne d'un polymère à motif éthylène α, β-dicarboxylique dont l'un des groupements carboxyliques a été amené à réagir avec une polyamine comportant un ou plusieurs groupements amine primaire ou secondaire. Les polymères fixant amphotères répondant à la définition donnée ci-dessus plus particulièrement préférés sont choisis parmi les polymères suivants :
1) les polymères résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537.
(2) les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

   Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl acrylamide, le N-tertiooctyl acrylamide, le N-octylacrylamide, octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou tumarique.
   Les comonomères basiques préférés sont des méthacrylates d'aminoéthyle, de butyl aminoéthyles, de N,N'-diméthylaminoéthyle, de N-tertiobutylaminoéthyle.
   On utilise particulièrement les copolymères dont la dénomination CTFA (4ème ED., 1991) est Octylacrylamide / acrylates / butylaminoethylmethacrylate copolymer tels que les produits vendus sous la dénomination AMPHOMER ou LOVOCRYL 47 par la société NATIONAL STARCH.
(3) les polyamino amides réticulés et alcoylés partiellement ou totalement dérivant de polyaminoamides de formule générale :

   ⁅CO-R₁₀-CO-Z⁆ (III)

   dans laquelle R₁₀ représente un radical divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical

      -NH⁅(CH₂)ₓ-NH⁆ₚ (IV)

      où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (IV) ci-dessus, dans lequel x=2 et p=1 et qui dérive de l'éthylènediamine, ou le radical dérivant de la pipérazine : 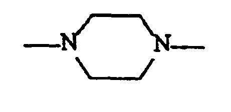
   c) dans les proportions de 0 à 20 moles % le radical -NH-(CH₂)₈-NH-dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,395 mole d'agent réticulant par groupement amine du polyaminoamide et alcoylés par action d'acide acrylique, d'acide chloracétique ou d'une alcane sulfone ou de leurs sels.

   Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
   Les alcanes sulfones utilisées dans l'alcoylation sont de préférence la propane ou la butane sulfone, les sels des agents d'alcoylation sont de préférence les sels de sodium ou de potassium.
(4) les polymères comportant des motifs zwittérioniques de formule : 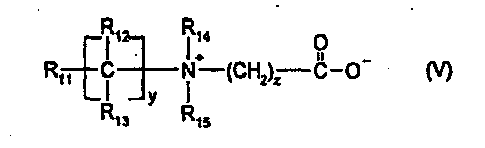 dans laquelle R₁₁ désigne un groupement insaturé polymérisable tel qu'un groupement acrylate, méthacrylate, acrylamide ou méthacrylamide, y et z représente un nombre entier de 1 à 3, R₁₂ et R₁₃ représentant un atome d'hydrogène, méthyle, éthyle ou propyle R₁₄ et R₁₅ représentent un atome d'hydrogène ou un radical alkyle de telle façon que la somme des atomes de carbone dans R₁₄ et R₁₅ ne dépasse pas 10.
   Les polymères comprenant de telles unités peuvent également comporter des motifs dérivés de monomères non zwittérioniques tels que l'acrylate ou méthacrylate de diméthyl ou diéthylamioéthyle ou des alkyle acrylates ou méthacrylates, des acrylamides ou méthacrylamides ou l'acétate de vinyle.
   A titre d'exemple, on peut citer le copolymère de méthacrylate de méthyle/diméthyl carboxyméthylammonio éthylméthacrylate de méthyle tel que le produit vendu sous la dénomination DIAFORMER Z301 par la société SANDOZ.
(5) les polymères dérivés du chitosane comportant des motifs monomères répondant aux formules suivantes : 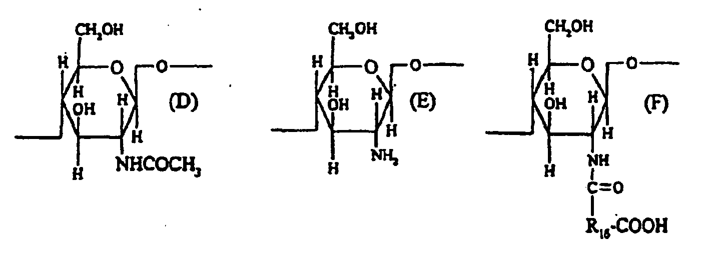 le motif D étant présent dans des proportions comprises entre 0 et 30%, le motif E dans des proportions comprises entre 5 et 50% et le motif F dans des proportions comprises entre 30 et 90%, étant entendu que dans ce motif F, R₁₆ représente un radical de formule : 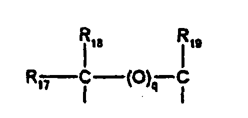 dans laquelle si q=0, R₁₇, R₁₈ et R₁₉, identiques ou différents, représentant chacun un atome d'hydrogène, un reste méthyle, hydroxyle, acétoxy ou amino, un reste mooalcoylamine ou un reste dialcoylamine éventuellement interrompus par un ou plusieurs atomes d'azote et/ou éventuellement substitués par un ou plusieurs groupes amine, hydroxyle, carboxyle, alcolylthio, sulfonique, un reste alcoylthio dont le groupe alcoyle porte un reste amino, l'un au moins des radicaux R₁₇, R₁₈ et R₁₉ étant dans ce cas un atome d'hydrogène ;
   ou si q=1, R₁₇, R₁₈ et R₁₉ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
(7) Les polymères répondant à la formule générale (VI) par exemple décrits dans le brevet français 1 400 366: 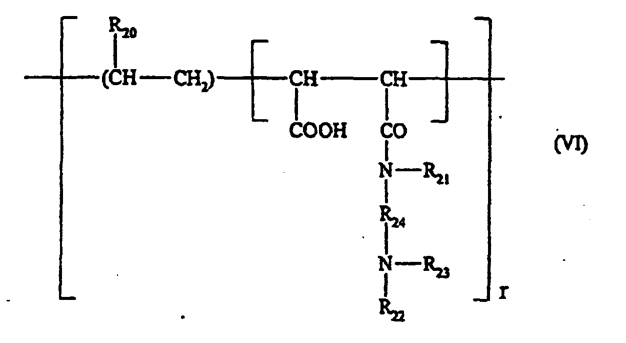 dans laquelle R₂₀ représente un atome d'hydrogène, un radical CH₃O, CH₃CH₂O, phényle, R₂₁ désigne l'hydrogène ou un radical alkyle inférieur tel que méthyle, éthyle, R₂₂ désigne l'hydrogène ou un radical alkyle inférieur tel que méthyle, éthyle, R₂₃ désigne un radical alkyle inférieur tel que méthyle, éthyle ou un radical répondant à la formule : -R₂₄-N(R₂₂)₂, R₂₄ représentant un groupement -CH₂-CH₂-, -CH₂-CH₂-CH₂-, -CH₂-CH(CH₃)-, R₂₂ ayant les signification mentionnées ci-dessus.
   ainsi que les homologues supérieurs de ces radicaux et contenant jusqu'à 6 atomes de carbone.
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- (VII)

      où D désigne un radical 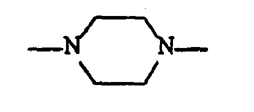 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne.
   b) Les polymères de formule :

      -D-X-D-X- (VII)

      où D désigne un radical 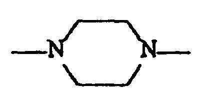 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) les copolymères alkyl(C1-C5)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères fixants amphotères particulièrement préférés selon l'invention sont ceux de la famille (3) tels que les copolymères dont la dénomination CTFA est Octylacrylamide/acrylates/butylaminoethylmethacrylate copolymer tels que les produits vendus sous les dénominations AMPHOMER, AMHOMER LV 71 ou LOVOCRYL 47 par la société NATIONAL STARCH et ceux de la famille (4) tels que les copolymère de méthacrylate de méthyle / diméthyl carboxyméthylammonio éthylméthacrylate de méthyle par exemple vendu sous la dénomination DIAFORMER Z301 par la société SANDOZ.

Les polymères fixants non ioniques utilisables selon la présente invention sont choisis par exemple parmi :
- les homopolymères de vinylpyrrolidone ;
- les copolymères de vinylpyrrolidone et d'acétate de vinyle ;
- les polyalkyloxazolines telles que les polyéthyloxazolines proposées par la société DOW CHEMICAL sous les dénominations PEOX 50 000, PEOX 200 000 et PEOX 500 000 ;
- les homopolymères d'acétate de vinyle tels que le produit proposé sous le nom de APPRETAN EM par la société HOECHST ou le produit proposé sous le nom de RHODOPAS A 012 par la société RHONE POULENC
- les copolymères d'acétate de vinyle et d'ester acrylique tels que le produit proposé sous le nom de RHODOPAS AD 310 de RHONE POULENC ;
- les copolymères d'acétate de vinyle et d'éthylène tels que le produit proposé sous le nom de APPRETAN TV par la société HOECHST ;
- les copolymères d'acétate de vinyle et d'ester maléïque par exemple de maléate de dibutyle tels que le produit proposé sous le nom de APPRETAN MB EXTRA par la société HOECHST;
- les copolymères de polyéthylène et d'anhydride maléïque ;
- les homopolymères d'acrylates d'alkyle et les homopolymères de méthacrylates d'alkyle tels que le produit proposé sous la dénomination MICROPEARL RQ 750 par la société MATSUMOTO ou le produit proposé sous la dénomination LUHYDRAN A 848 S par la société BASF ;
- les copolymères d'esters acryliques tels que par exemple les copolymères d'acrylates d'alkyle et de méthacrylates d'alkyles tels que les produits proposés par la société ROHM&HAAS sous les dénominations PRIMAL AC-261 K et EUDRAGIT NE 30 D, par la société BASF sous les dénominations ACRONAL 601, LUHYDRAN LR 8833 ou 8845, par la société HOECHST sous les dénominations APPRETAN N 9213 ou N9212;
- les copolymères d'acrylonitrile et d'un monomère non ionique choisi par exemple parmi le butadiène et les (méth)acrylates d'alkyle ; on peut citer les produits proposés sous les dénominations NIPOL LX 531 B par la sodété NIPPON ZEON ou ceux proposés sous la dénomination CJ 0601 B par la société ROHM & HAAS ;
- les polyuréthannes tels que les produits proposés sous les dénominations ACRYSOL RM 1020 ou ACRYSOL RM 2020 par la société ROHM & HAAS, les produits URAFLEX XP 401 UZ, URAFLEX XP 402 UZ par la société DSM RESINS ;
- les copolymères d'acrylate d'alkyle et d'uréthanne tels que le produit 8538-33 par la société NATIONAL STARCH ;
- les polyamides tels que le produit ESTAPOR LO 11 proposé par la société RHONE POULENC.
- les gommes de guar non ioniques chimiquement modifiées ou non modifiées.
   Les gommes de guar non ioniques non modifiées sont par exemple les produits vendus sous la dénomination VIDOGUM GH 175 par la société UNIPECTINE et sous la dénomination JAGUAR C par la société MEYHALL. Les gommes de guar non-ioniques modifiées utilisables selon l'invention sont de préférence modifiées par des groupements hydroxyalkyle en C₁-C₆. On peut mentionner à titre d'exemple, les groupements hydroxyméthyle, hydroxyéthyle, hydoroxypropyle et hydroxybutyle.
   Ces gommes de guar sont bien connues de l'état de la technique et peuvent par exemple être préparées en faisant réagir des oxydes d'alcènes correspondants, tels que par exemple des oxydes de propylène, avec la gomme de guar de façon à obtenir une gomme de guar modifiée par des groupements hydroxypropyle.
   De telles gommes de guar non-ioniques éventuellement modifiées par des groupements hydroxyalkyle sont par exemple vendues sous les dénominations commerciales JAGUAR HP8, JAGUAR HP60 et JAGUAR HP120. JAGUAR DC 293 et JAGUAR HP 105 par la société MEYHALL, ou sous la dénomination GALACTASOL 4H4FD2 par la société AQUALON.
   Les radicaux alkyle des polymères non ioniques ont de 1 à 6 atomes de carbone sauf mention contraire.
   Selon l'invention, on peut également utiliser les polymères fixants de type siliconés greffés comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-0 412 704, EP-A-0 412 707, EP-A-0 640 105 et WO 95/00578, EP-A-0582 152 et WO 93/23009 et les brevets US 4,693,935, US 4,728,571 et US 4,972,037. Ces polymères sont de préférence anioniques ou non ioniques. De tels polymères sont par exemple les copolymères susceptibles d'être obtenus par polymérisation radicalaire à partir du mélange de monomères constitué par :
   a) 50 à 90% en poids d'acrylate de tertiobutyle ;
   b) 0 à 40% en poids d'acide acrylique ;
   c) 5 à 40% en poids de macromère silicone de formule : 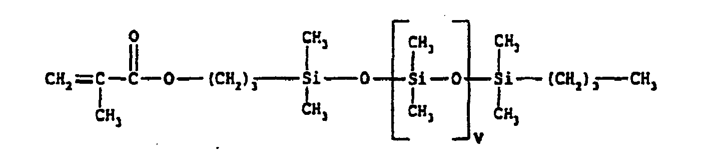 avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon la présente invention, les polymères fixants sont de préférence des polymères anioniques ou amphotères.

Les polymères fixants anioniques ou amphotères peuvent être si nécessaire neutralisés partiellement ou totalement. Les agents de neutralisation sont par exemple la soude, la potasse, l'amino-2-méthyl-2 propanol-1, la monoéthanolamine, la triéthanolamine ou la triisopropanolamine, les acides minéraux ou organiques tels que l'acide chlorhydrique ou l'acide citrique.

Le ou les polymères fixants sont par exemple présentes dans des concentrations comprises entre 0,05% et 20% en poids, et de préférence dans des concentrations comprises entre 0,1% et 10% en poids par rapport au poids total de la composition.

Le ou les amidons amphotères peuvent être présents dans des concentrations comprises entre 0,01% et 15% en poids, et de préférence dans des concentrations comprises entre 0.05% et 10% en poids et encore plus particulièrement de 0,1 à 5% en poids par rapport au poids total de la composition.

Le milieu cosmétiquement acceptable est de préférence constitué par de l'eau ou un mélange d'eau et de solvants cosmétiquement acceptables tels que des monoalcools, des polyalcools, des éthers de glycol ou des esters d'acides gras, qui peuvent être utilisés seuls ou en mélange. Ces solvants de préférence des alcools en C₁-C₆.

Parmi ces alcools, on peut citer l'éthanol, l'isopropanol, les polyalcools tels que le diéthylèneglycol, les éthers de glycol, tels que les monoalkyléthers de glycol, de diéthylèneglycol, de propylèneglycol ou de dipropylèneglycol. L'éthanol est particulièrement préféré.

La composition utilisée selon l'invention peut également contenir au moins un additif choisi parmi les épaississants, les esters d'acides gras, les esters d'acides gras et de glycérol, les silicones volatiles ou non volatiles, solubles ou insolubles dans la composition, les tensioactifs, les parfums, les conservateurs, les filtres solaires, les protéines, les vitamines, les polymères, les huiles végétales, animales, minérales ou synthétiques et tout autre additif classiquement utilisé dans les compositions cosmétiques pour les fibres kératiniques.

Ces additifs sont présents dans la composition utilisée selon l'invention dans des proportions pouvant aller de 0 à 20% en poids par rapport au poids total de la composition. La quantité précise de chaque additif est fonction de sa nature et est déterminée facilement par l'homme de l'art.

Bien entendu, l'homme du métier veillera à choisir le ou les éventuels composés à ajouter à la composition utilisée selon l'invention de manière telle que les propriétés avantageuses attachées intrinsèquement à la composition conforme à l'invention ne soient pas, ou substantiellement pas, altérées par l'addition envisagée.

En particulier, les compositions utilisées selon l'invention comprennent, de préférence, moins de 10 % en poids par rapport au poids total de la composition d'esters d'acides gras en C₈-C₃₀. Ainsi, les fibres kératiniques traitées avec les compositions selon l'invention n'ont pas un toucher, ni un aspect gras et le pouvoir fixant de la composition n'est pas diminué.

Les compositions utilisées selon l'invention peuvent se présenter sous forme de lait, de crème, de lotion épaissie ou non.

Les compositions utilisées selon l'invention peuvent être rincées et de préférence non-rincées pour la fixation de la coiffure ou la mise en forme des cheveux.

Elles sont plus particulièrement des produits de coiffage tels que des compositions de fixation (laques) et de coiffage. Les lotions peuvent être conditionnées sous diverses formes notamment dans des vaporisateurs, des flacons pompes ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux.

Lorsque la composition selon l'invention est conditionnée sous forme d'aérosol en vue d'obtenir une laque ou une mousse aérosol, elle comprend au moins un agent propulseur qui peut être choisi parmi les hydrocarbures volatils tels que le n-butane, le propane, l'isobutane, le pentane, un hydrocarbure chloré et/ou fluoré et leurs mélanges. On peut également utiliser en tant qu'agent propulseur le gaz carbonique, le protoxyde d'azote, le diméthyléther, l'azote, l'air comprimé et leurs mélanges.

La préparation des compositions selon l'invention est réalisée selon des méthodes bien connues de l'état de la technique. En particulier, les ingrédients sont mélangés puis conditionnés dans un récipient approprié selon l'utilisation envisagée.

L'invention va être maintenant plus complètement illustrée à l'aide des exemples suivants qui ne sauraient être considérés comme la limitant aux modes de réalisation décrits. (Dans ce qui suit MA signifie Matière Active).

### EXEMPLE 1

On a préparé trois lotions de mise en plis de composition suivante :

| FORMULATION TESTEE | A (Invention) | B (Comparatif) | C (Comparatif |
|---|---|---|---|
| AMIDON⁻¹ | 0.5 g | 1 g | - |
| Polymère fixant⁻² | 0.5 g | - | 1 g |
| Eau qsp | 100 g | 100 g | 100 g |
| Pouvoir fixant | 50 | 40 | 45 |

| | | | |
|---|---|---|---|
| ^{-1.}Amidon modifié par de l'acide 2-chloroéthyl aminodipropionique proposé par la société NATIONAL STARCH ^{-2.}Terpolymère de vinylpyrrolidone/acide acrylique/méthacrylate de lauryle vendu sous la dénomination ACRYLIDONE LM par la société ISP | | | |

On a appliqué chacune de ces composition sur des cheveux lavés et séchés, puis, on a demandé à un panel de 5 testeurs expérimentés d'évaluer le pouvoir fixant de chaque composition. La notation va de 0 (aucun pouvoir fixant) à 50 (excellent pouvoir fixant).

La composition A présente un pouvoir fixant supérieur à celui des compositions B et C qui ne contiennent qu'un des deux composés de l'invention.

Les cheveux traités avec la composition (A) selon l'invention présente également de bonnes propriétés de toucher.

### EXEMPLE 2

On a préparé trois lotions de mise en plis de composition suivante :

| FORMULATION TESTEE | A(Invention) | B(Comparatif) | C(Comparatif) |
|---|---|---|---|
| AMIDON⁻¹ | 0,5 g | 1 g | - |
| Polymère fixant⁻³ | 0,5 g | - | 1g |
| Eau qsp | 100 g | 100 g | 100 g |
| Pouvoir fixant | 35 | 30 | 30 |

| | | | |
|---|---|---|---|
| ⁻¹- Amidon modifié par de l'acide 2-choloroéthyl aminodipropionique proposé par la société NATIONAL STARCH ⁻³- Terpolymère de acrylamide / acide acrylique / Chlorure de diméthyl diallyl ammonium en solution aqueuse à 10% vendu sous la dénomination MERQUAT PLUS 3330 par la société GALGO | | | |

On a appliqué chacune de ces compositions sur des cheveux faiblement décolorés lavés et séchés, puis, on a demandé à un panel de 5 testeurs expérimentés d'évaluer le pouvoir fixant de chaque composition. La notation va de 0 (aucun pouvoir fixant) à 50 (excellent pouvoir fixant).

La composition A présent un pouvoir fixant supérieur à celui des compostion B et C qui ne contiennent qu'un des deux composés de l'invention. Les cheveux traités avec la composition (A) selon l'invention présente également de bonnes propriétés de toucher, de douceur et de démêlage.

### EXEMPLE 3

On a préparé une composition de spray fixant conditionnée dans un flacon-pompe de composition suivante :

| | |
|---|---|
| - Amidon modifié par de l'acide 2-chloroéthyl aminodipropionique | 0,8 g |
| | |
| - Terpolymère de acrylamide / acide acrylique /chlorure de diméthyl diallyl ammonium en solution aqueuse à 10% vendu sous la dénomination MERQUAT PLUS 3330 par la société CALGON | 0,2 gMA |
| | |
| -Polyquaternium-37 (nom INCI) vendu sous la dénomination SALCARE SC 95 par la société ALLIED COLLOID | 0,6 gMA |
| | |
| - Eau qsp | 100 g |

La composition présente les mêmes propriétés que celles de l'exemple 1.

### EXEMPLE 4

On a préparé une composition gel de coiffage de composition suivante :

| | |
|---|---|
| - Amidon modifié par de l'acide 2-chloroéthyl aminodipropionique | 0,5 g |
| | |
| - Terpolymère de acrylamide / acide acrylique / chlorure de diméthyl diallyl ammonium en solution aqueuse à 10% vendu sous la dénomination MERQUAT PLUS 3330 par la société CALGON | 0,5 gMA |
| | |
| - Acide polyacrylique réticulé vendu sous la dénomination SYNTHALEN K par la société 3V | 0.6 gMA |
| | |
| - Ethanol à 95° | 8,5 g |
| | |
| - Triéthanolamine qs | pH 7,5 |
| - Eau qsp | 100 g |

La composition est appliquée sur des cheveux lavés et essorés. Elle conduit à un bon maintien de la coiffure et à de bonnes propriétés de démêlage et de toucher.

### EXEMPLE 5

On a préparé un lait fixant conditionné dans un flacon-pompe de composition suivante :

| | |
|---|---|
| - Terpolymère de vinylpyrrolidone / acide acrylique / méthacrylate de lauryle vendu sous la dénomination ACRYLIDONE LM par la société ISP | 2 gMA |
| | |
| - Amino-2 méthyl-2 propanol-1 | 0,6 g |
| | |
| - Amidon modifié par de l'acide 2-chloroéthyl aminodipropionique | 0,5 g |
| | |
| - Eau qsp | 100 g |

La composition est appliquée sur des cheveux lavés et essorés. Elle conduit à un bon maintien de la coiffure et à de bonnes propriétés de démêlage et de toucher.

### EXEMPLE 6

On a préparé une composition gel de soin coiffant de composition suivante :

| | |
|---|---|
| - Amidon modifié par de l'acide 2-chloroéthyl aminodipropionique | 0,5 g |
| | |
| - Copolymère hydroxyéthylcellulose/chlorure de diallyl diméthyl ammonium vendu sous la dénomination CELQUAT L 200 par la société NATIONAL STARCH | 0,3 gMA |
| | |
| - Gomme d'hydroxypropylguar vendu par la société RHONE POULENC sous la dénomination JAGUAR HP 105 | 0,3 g |
| | |
| - Acide polyacrylique réticulé vendu sous la dénomination SYNTHALEN K par la société 3 V | 0,4 gMA |
| | |
| - Ethanol à 95° | 8,5 g |
| | |
| - Amino-2 méthyl-2 propanoYl-1 qs | pH 7,5 |
| | |
| - Eau qsp | 100 g |

La composition est appliquée sur des cheveux lavés et essorés. Elle conduit à un bon maintien de la coiffure et à de bonnes propriétés de démêlage et de toucher.

## Revendications

1. Utilisation d'une composition cosmétique, **caractérisée en ce qu'**elle comprend, dans un milieu cosmétiquement acceptable, au moins un polymère fixant et au moins un amidon amphotère ;
pour la fixation ou la mise en forme des cheveux.

2. Utilisation selon la revendication précédente, **caractérisée en ce que** le ou les polymères fixants sont présents dans des concentrations comprises entre 0,05 et 20% en poids, et de préférence dans des concentrations comprises entre 0,1 et 10%, en poids par rapport au poids total de la composition.

3. Utilisation selon l'une des revendications précédentes, **caractérisée en ce que** l'amidon amphotère est présent dans des concentrations comprises entre 0,01% et 15% en poids par rapport au poids total de la composition, et de préférence comprises entre 0,05% et 10% en poids par rapport au poids total de la composition.

4. Utilisation selon l'une quelconque des revendications précédentes, **caractérisée par le fait que** le polymère fixant est choisi parmi les polymères fixants anioniques, cationiques, amphotères, non ioniques et leurs mélanges.

5. Utilisation selon l'une quelconque des revendications précédentes, **caractérisée en ce que** l'amidon amphotère est choisi parmi les composés de formules (I) à (IV) : 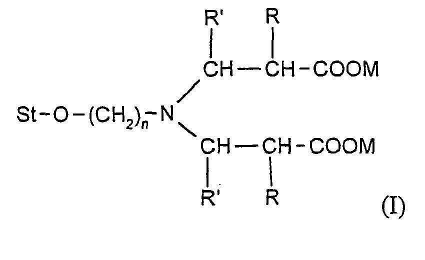 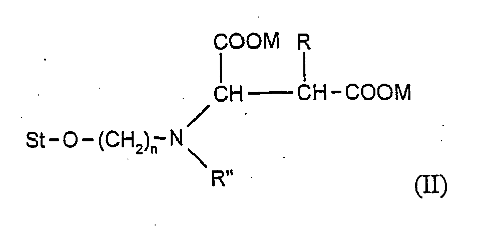 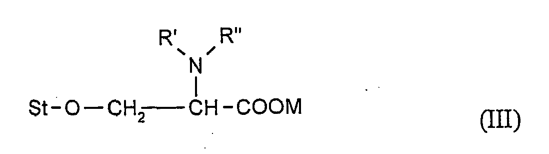 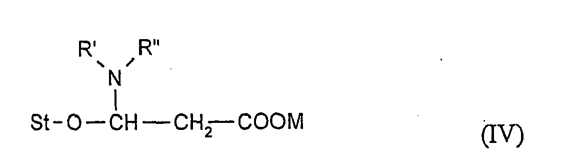 formules dans lesquelles :
St-O représente une molécule d'amidon,
R, identique ou différent, représente un atome d'hydrogène ou un radical méthyle,
R', identique ou différent, représente un atome d'hydrogène, un radical méthyle ou un groupement -COOH,
n est un entier égal à 2 ou 3,
M, identique ou différent, désigne un atome d'hydrogène, un métal alcalin ou alcalinoterreux, NH₄ ou une amine organique,
R" représente un atome d'hydrogène ou un radical alkyle ayant de 1 à 18 atomes de carbone.

6. Utilisation selon la revendication précédente, **caractérisée par le fait que** les amidons sont de formules (I) ou (II).

7. Utilisation selon la revendication précédente, **caractérisée par le fait que** R, R', R" et M représentent un atome d'hydrogène et n est égal à 2.

8. Utilisation selon l'une quelconque des revendications précédentes, **caractérisée en ce que** le milieu cosmétiquement acceptable comprend de l'eau ou un mélange d'eau et d'au moins un solvant cosmétiquement acceptable.

## Claims

1. Use of a cosmetic composition, **characterized in that** it comprises, in a cosmetically acceptable medium, at least one fixing polymer and at least one amphoteric starch;
for the fixing or shaping of the hair.

2. Use according to the preceding claim, **characterized in that** the fixing polymer or polymers are present in concentrations of between 0.05 and 20% by weight and preferably in concentrations of between 0.1 and 10% by weight, with respect to the total weight of the composition.

3. Use according to either of the preceding claims, **characterized in that** the amphoteric starch is present in concentrations of between 0.01% and 15% by weight, with respect to the total weight of the composition, and preferably of between 0.05% and 10% by weight, with respect to the total weight of the composition.

4. Use according to any one of the preceding claims, **characterized in that** the fixing polymer is chosen from anionic, cationic, amphoteric or nonionic fixing polymers and their blends.

5. Use according to any one of the preceding claims, **characterized in that** the amphoteric starch is chosen from the compounds of formulae (I) to (IV): 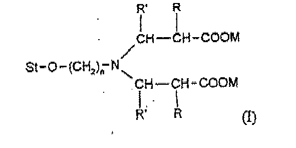 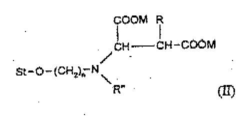 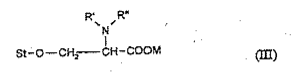 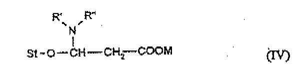 in which formulae:
St-O represents a starch molecule,
R, which is identical or different, represents a hydrogen atom or a methyl radical,
R', which is identical or different, represents a hydrogen atom, a methyl radical or a -COOH group,
n is an integer equal to 2 or 3,
M, which is identical or different, denotes a hydrogen atom, an alkali or alkaline earth metal, NH₄ or an organic amine,
R" represents a hydrogen atom or an alkyl radical having from 1 to 18 carbon atoms.

6. Use according to the preceding claim, **characterized in that** the starches are of formula (I) or (II).

7. Use according to the preceding claim, **characterized in that** R, R', R" and M represent a hydrogen atom and n is equal to 2.

8. Use according to any one of the preceding claims, **characterized in that** the cosmetically acceptable medium comprises water or a mixture of water and of at least one cosmetically acceptable solvent.

## Patentansprüche

1. Verwendung einer kosmetischen Zusammensetzung für die Fixierung oder Formgebung der Haare, **dadurch gekennzeichnet, dass** sie in einem kosmetisch akzeptablen Medium mindestens ein fixierendes Polymer und mindestens eine amphotere Stärke enthält.

2. Verwendung nach dem vorhergehenden Anspruch, **dadurch gekennzeichnet, dass** das oder die fixierenden Polymere in Konzentrationen im Bereich von 0,05 bis 20 Gew.-% und vorzugsweise in Konzentrationen im Bereich von 0,1 bis 10 Gew.-% vorliegen.

3. Verwendung nach einem der vorhergehenden Ansprüche, **dadurch gekennzeichnet, dass** die amphotere Stärkeverbindung in Konzentrationen im Bereich von 0,01 bis 15 Gew.-%, bezogen auf das Gesamtgewicht der Zusammensetzung, und vorzugsweise in Konzentrationen im Bereich von 0,05 bis 10 Gew.-%, bezogen auf das Gesamtgewicht der Zusammensetzung, enthalten ist.

4. Verwendung nach einem der vorhergehenden Ansprüche, **dadurch gekennzeichnet, dass** das fixierende Polymer unter den anionischen, kationischen, amphoteren, nichtionischen fixieren den Polymeren oder deren Gemischen ausgewählt ist.

5. Verwendung nach einem der vorhergehenden Ansprüche, **dadurch gekennzeichnet, dass** die amphotere Stärkeverbindung unter den Verbindungen der folgenden Formeln (I) bis (IV) ausgewählt ist: 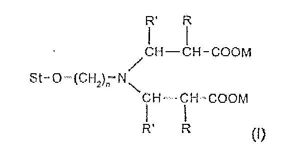 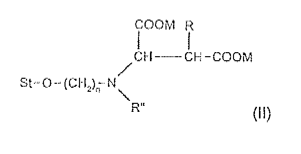 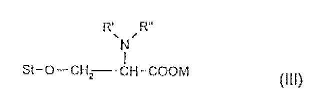 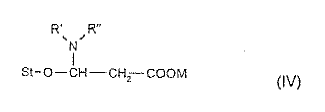 wobei in den Formeln bedeuten:
· St-O ein Stärkemolekül,
· die Gruppen R, die gleich oder verschieden sind, ein Wasserstoffatom oder die Methylgruppe,
· die Gruppen R', die gleich oder verschieden sind, ein Wasserstoffatom, die Methylgruppe oder die Gruppe -COOH,
· n 2 oder 3,
· die Gruppen M, die gleich oder verschieden sind, ein Wasserstoffatom, ein Alkali- oder Erdalkalimetall, NH₄ oder ein organisches Amin,
· die Gruppen R" ein Wasserstoffatom oder eine Alkylgruppe mit 1 bis 18 Kohlenstoffatomen.

6. Verwendung nach dem vorhergehenden Anspruch, **dadurch gekennzeichnet, dass** es sich bei der Stärkeverbindung um eine Verbindung der Formel (I) oder (II) handelt.

7. Verwendung nach der vorhergehenden Anspruch, **dadurch gekennzeichnet, dass** R, R', R" und M ein Wasserstoffatom bedeuten und n 2 ist.

8. Verwendung nach einem der vorhergehenden Ansprüche, **dadurch gekennzeichnet, dass** das kosmetisch akzeptable Medium Wasser oder ein Gemisch aus Wasser und mindestens einem kosmetisch akzeptablen Lösungsmittel enthält.
